# EUROPÄISCHE PATENTSCHRIFT

(11) **EP 3 478 196 B1**
(45) Veröffentlichungstag und Bekanntmachung des Hinweises auf die Patenterteilung: **27.04.2022**
(21) Anmeldenummer: 17737526.8
(22) Anmeldetag: 03.07.2017
(51) Int. Cl.: A61B 17/86, A61B 17/88

(54) **IMPLANTATIONSHILFSMITTEL FÜR DIE NUTZUNG VON OBERFLÄCHENSENSITIVEN IMPLANTATEN**
IMPLANTATION AID FOR THE USE OF SURFACE-SENSITIVE IMPLANTS
AUXILIAIRE D'IMPLANTATION POUR L'UTILISATION D'IMPLANTS SENSIBLES EN SURFACE

(30) Priorität: 04.07.2016 DE 102016112154
(43) Veröffentlichungstag der Anmeldung: 08.05.2019
(73) Patentinhaber: Karl Leibinger Medizintechnik GmbH & Co. KG, 78570 Mühlheim (DE)
(72) Erfinder: AKSU, Adem, 78054 VS-Schwenningen (DE); REINAUER, Frank, 78576 Emmingen-Liptingen (DE); WOLFRAM, Tobias, 63303 Dreieich (DE)
(74) Vertreter: Winter, Brandl - Partnerschaft mbB
(86) Internationale Anmeldenummer: PCT/EP2017/066479
(87) Internationale Veröffentlichungsnummer: WO 2018/007308

(56) Entgegenhaltungen:
- WO-A1-2011/080104
- WO-A2-2005/053753
- DE-U1-202011 107 522
- US-A1- 2003 054 318
- US-A1- 2004 254 576
- US-A1- 2013 218 214
- US-B1- 6 330 845

## Beschreibung

Die Erfindung betrifft ein Implantationsset nach dem Oberbegriff des Anspruchs 1 mit einem Werkzeug und zumindest einem dazu passenden Knochenkontaktierungsorgan, das als eine Vorrichtung zum direkten Kontaktieren eines Knochens ausgelegt ist, insbesondere ein solches Set, welches das Ein-, Aus- und Anbringen von Implantaten aus Werkstoffen mit sensiblen Oberflächenmodifikationen und/oder Beschichtungen mit möglichst kleiner oder keiner Beschädigung des Implantates ermöglicht. Dies kann zum Beispiel mit einer medizinischen Werkzeug - Schraubenkombination erreicht werden, bei der ein Schraubendreher der als Werkzeug agiert, und zumindest einer zum Schraubendreher passenden Schraube, die zum Eindrehen in einen Knochen, etwa eines Säugers, wie eines Menschen oder eines Säugetiers geeignet ist, wobei die Schraube einerseits einen Schraubenkopf und andererseits ein Gewinde besitzt, wobei der Schraubenkopf eine Werkzeugaufnahme besitzt, die geometrisch und oberflächentopographisch an die Außenkontur einer Spitze des Schraubendrehers angepasst ist.

Aus dem Stand der Technik sind bereits Schrauben und Schraubendreher bekannt, die zum Einsatz in der Medizintechnik vorgesehen sind. Üblicherweise sind aber die Werkzeuge härter, als die Schrauben. Das hat die negative Auswirkung, dass beim Einschrauben der Schraube eine Beschädigung des Schraubenkopfes, insbesondere im Kontaktierbereich mit dem Werkzeug, eine Beschädigung auftreten kann. Auch oberflächennahe Beschädigungen von beschichteten Implantaten oder Oberflächenmodifizierten Implantaten während des An- und Einbringens sind bekannt und können letztendlich zum Versagen des Implantates führen. Zusätzlich können bei der Herausnahme von Implantaten, z.B. einer Schraube, dann bedingt durch die zuvor zugeführten Beschädigungen, Probleme auftreten. Dies kann sogar so schwerwiegend sein, dass die Schraube im Körper verbleiben muss, obwohl dies medizinisch nicht angezeigt ist. Beispielhafter Stand der Technik ist aus der DE 20 2013 105 409 U1, der DE 20 2004 004 844 U1 sowie der US 2011/ 0 015 682 A1 bekannt. Weiterer Stand der Technik ist auch aus der US 2003/0054318 A1 und der US 2013/0218214 A1 bekannt.

Im klassischen Maschinenbau ist es jedoch bevorzugt ein hartes Werkzeug zu besitzen und eine dazu weichere Schraube einzusetzen. Dies liegt daran, dass im Beschädigungsfall lediglich die Schraube zu ersetzen ist, nicht aber das wesentlich teurere Werkzeug.

Es ist die Aufgabe der vorliegenden Erfindung aber gerade für den Medizintechnikbereich eine bessere Lösung zur Verfügung zu stellen, die gerade das in jüngerer Zeit notwendig gewordene Anbringen, Einbringen und Ausbringen von innovativen Implantaten aus neuen Werkstoffen mit speziellen Oberflächenbeschichtungen und Modifikationen ermöglichen soll, ohne Beschädigung des Implantates selbst zu verursachen. Auch das Wiederlösen von Schrauben und Wiedereindrehen nach einem Lösevorgang, soll mithilfe der in genannten Instrumente und Werkzeuge erleichtert werden.

Diese Aufgabe wird verblüffend einfach durch den Gegenstand des Anspruchs 1 gelöst.

Zum einen ist bei der Umsetzung einer Schraubendreher-Schrauben-bezogenen Lösung die Aufgabe dadurch gelöst, dass der Schraubenkopf zumindest im Bereich der Werkzeugaufnahme eine größere Härte besitzt als zumindest der zum Kontaktieren der Werkzeugaufnahme vorgesehene Teil der Spitze des Schraubendrehers. Anders, als bisher in jedem Lehrbuch für einen Ingenieur gelehrt, wird nun genau entgegen gehandelt und die grundsätzlich wesentlich Schraube weicher als der Schraubendreher ausgestaltet. Was im normalen Maschinenbaubereich verwerflich ist, bietet sich aber im Medizintechnikbereich, gerade bei den dort vorliegenden Problematiken an. Die allgemein niedrige Härte der Implantate (inklusive Schrauben) kommt von den verwendeten Werkstoffen und den Oberflächenveränderungen. Diese habe bessere Eigenschaften in Bezug auf medizinisch/biologisch relevante Aspekte, zeichnen sich aber mechanisch durch einen Verlust an Härte im Vergleich zu den traditionell unbeschichteten Implantaten aus Titan oder Edelstahl, aus.

Es wird also eine für den Medizintechnikbereich adäquate und doch einfach umzusetzende Lösung vorgestellt, die gerade bei Hilfsmitteln, Werkzeugen und strukturierten Vorrichtungen für das Einbringen, das Anbringen und das Fixieren von Implantaten insbesondere aus biodegradierbaren Metallen und deren Legierungen sowie resorboierbare bzw. nicht resorbierbare Polymere, Keramiken und deren Komposite, unschätzbare Vorteile bietet. Zusätzlich bieten sich dadurch Lösungen für den Bereich der klassischen Werkstoffe für Implantate, wie z.B. Titan oder Edelstahl an, deren Oberflächen mechanisch, chemisch oder physikalisch optimiert und strukturiert wurden, wobei aber deutlich sensiblere Oberflächen geschaffen wurden. Es werden also gerade Lösungen für Implanatationshilfsmittel, die zum Anbringen, Einbringen und Ausbringen von Implantaten (zum Beispiel einer Schraube oder Platte) aus biodegradierbaren oder nicht biodegradierbaren Metallen und Legierungen sowie resorbierbare bzw. nicht resorbierbare Polymere, Keramiken und deren Komposite vorgesehen sind, erreicht.

Ein Selbsthalteeffekt wird zum anderen dadurch hergestellt, indem alle zum Kontaktieren der Schraubendreherspitzenkontaktierflächen der Schraube vorgesehene Werkzeugaufnahmekontaktflächen des Schraubendrehers zwischen ca. 2,5° bis ca. 7,5° zur Längsachse des Schraubendrehers bzw. zu dessen Rotationsachse geneigt sind.

Vorteilhafte Ausführungsformen sind in den Unteransprüchen beansprucht und werden nachfolgend näher erläutert.

So ist es von Vorteil, wenn die Werkzeugaufnahme als Vertiefung ausgebildet ist, da dann der Schraubendreher einfach in die Vertiefung mit seiner Spitze eindringen kann und selbst bei leichtem Winkelversatz der Längsachse des Schraubendrehers zur Längsachse der Schraube, bspw. um bis zu ca. 15° bis ca. 20° noch ein Anziehen der Schraube ohne Auftretens eines Zerstörens der Werkzeugaufnahme stattfinden kann.

Um die Fertigung kostengünstig und universell einsetzbar zu halten hat es sich bewährt, wenn die Werkzeugaufnahme einen Schlitz- oder eine Kreuzschlitz- oder eine Inbus- oder eine Torx-Geometrie besitzt und der Schraubendreher an der Spitze dazu kongruent / passend ausgebildet ist.

Um einen Selbsthalteeffekt hervorrufen zu können, ist es von Vorteil, wenn die, etwa als Vertiefung ausgebildete Werkzeugaufnahme (exakt) in Längsrichtung der Schraube ausgerichtete Schraubendreherspitzenkontaktierflächen besitzt, wohingegen Werkzeugaufnahmekontaktflächen an der Spitze des Schraubendrehers zur Längsrichtung der Schraube (und/oder des Schraubendrehers) geneigt (/ schräg / quer /angestellt) ausgerichtet sind.

Der Selbsthalteeffekt lässt sich besonders gut herstellen, wenn alle zum Kontaktieren der Schraubendreherspitzenkontaktierflächen der Schraube vorgesehene Werkzeugaufnahmekontaktflächen des Schraubendrehers um ca. 5° +/- 0,25° zur Längsachse des Schraubendrehers bzw. zu dessen Rotationsachse geneigt sind.

Ein vorteilhaftes Ausführungsbeispiel ist auch dadurch gekennzeichnet, dass die Geometrien und die Ausmaße der Vertiefung im Schraubenkopf der als Vorsprung ausgebildeten Spitze des Schraubendrehers so aufeinander angepasst sind, dass ein Selbsthalten der Schraube auf der Spitze beim Eingreifen der Spitze in die Vertiefung erzwungen ist, etwa durch Erzeugung einer (Press-)Passung.

Um die Auffädelwirkung eines Drahtes nutzen zu können, ist es von Vorteil, wenn die Schraube und der Schraubendreher jeweils ein vorzugsweise zentrisch angeordnetes Durchgangsloch besitzen. Es ist somit sowohl in der Schraube als auch im Schraubendreher je ein Durchgangsloch enthalten. Ein Draht kann dann einfach in den Knochen des Patienten gepickt werden, um den Draht herum kann dann ein über den Draht geschobener Bohrer in den Knochen hinein bohren, danach, insbesondere nach Entnahme des Bohrers, kann eine über den Draht geführte Schraube mittels eines ebenfalls über den Draht geführten Schraubendrehers in den Knochen weiter eingeschraubt werden. Natürlich ist der Draht auch nach der Bohrung oder nach dem Einsetzen der Schraube in die Bohrung entnehmbar.

Es ist zweckmäßig, wenn die Durchgangslöcher der Schraube und des Schraubendrehers im gegenseitigen Kontaktbereich im Querschnitt kreisrund und gleich groß bemessen ausgebildet sind. Ein Verhaken des Drahtes kann dann verhindert werden. Die Führung ist verbessert.

Um auf herkömmliche Drahtkonfigurationen zurückgreifen zu können und ein gutes Reinigen des Schraubendrehers zu ermöglichen, ist es von Vorteil, wenn das Durchgangsloch im Schraubendreher mehrfach, etwa zweifach oder dreifach, gestuft ausgebildet ist, wobei ein kleinster Durchmesser ca. 0,6 mm, ein mittlerer Durchmesser ca. 0,8 mm und ein größerer Durchmesser ca. 1,0 mm beträgt.

Dabei ist es von Vorteil, wenn eine Stufe des Durchgangsloches des Schraubendrehers in einem sich von einem Griffbereich zur Spitze erstreckenden Schaftbereich, vorzugsweise im vorderen, distalseitigen Drittel des Schafts, vorhanden ist.

Ferner ist es von Vorteil, wenn der Schraubendreher als integrales, einmaterialiges Bauteil, etwa aus Kunststoff, bspw. Kunststoffspritzgussmaterial, einem Metall, wie einer Eisen-, Leichtmetall- oder Titanlegierung bestehend ausgebildet ist, oder der Schraubendreher mehrteilig und/oder mehrmaterialig aufgebaut ist. Gerade im letztgenannten Fall lässt sich ein Auswechseln von einzelnen Bestandteilen des Schraubendrehers einfach bewerkstelligen, sodass ein Reinigen und Instandsetzen erleichtert ist.

Gerade bei der Mehrteilig und/oder Mehrmaterialigkeit, ist es von Vorteil, wenn der Griff aus Kunststoff, wie Silikon oder einem Spritzgussmaterial besteht, der Schaft aus Metall, wie Eisen, oder Leichtmetall oder Titan besteht und/oder die Spitze Kunststoff, bspw. ein Polymer oder einen faserverstärkten Kunststoff oder Leichtmetall oder Keramik aufweist / daraus besteht oder die Spitze eine keramische Beschichtung auf der Außenseite besitzt.

Wenn die Spitze wechselbar am Griff angekoppelt ist, etwa mittels des Schaftes, so kann sich die Instandhaltung auf das mit der Schraube interagierende Bauteil beschränken.

Es ist von Vorteil, wenn der Schraubendreher als Drehmomentschlüssel ausgebildet ist, da dadurch ein Überdrehen der Schraube wirksam verhindert werden kann.

Auch die Schraube kann aus beschichtetem Material bestehen oder beschichtetes Metall besitzen, bspw. eine Keramikbeschichtung. Des Weiteren besitzen auch andere Implantate, wie z.B. Platten, "Meshes" oder "Scaffolds" Beschichtungen. Diese Beschichtungen oder Oberflächenmodifikationen werden beim Anpassen (Anbringen) oder Einbringen der Implantate häufig beschädigt, so dass während der Interaktion von Implantatoberfläche mit Geweben unspezifische Reaktionen an den beschädigten Stellen auftreten können, wie z.B. chemische Korrosion, materieller Abbau oder chemische Degradation. Dies soll durch die erwähnten Veränderungen für alle Instrumente, die direkten Kontakt mit den Implantaten während des Anbringens, Einbringen und auch Ausbringen, verhindert werden.

Dabei hat es sich bewährt, wenn die Schraube und/oder die Spitze des Schraubendrehers PVD- oder CVD-beschichtet ist. Zusätzlich können die Werkzeuge und Instrumente auch spezifisch an der Oberfläche modifiziert werden, z.B. kann eine µm-Rauhigkeit eingesetzt werden, um nachfolgenden polymeren Oberflächenschutzschichten eine verbesserte Untergrundhaftung zu vermitteln, damit diese Schicht zwar weich ist, aber stabil auf dem Instrument, z.B. dem Schraubendreher oder dem Halterungswerkzeug verbleibt. Bei diesen modifizierten Instrumenten/Werkezeugen wird zunächst eine µm-Topograhpie z.B. durch, physikalische, chemische oder mechanische Verfahren aufgebracht, wie z.B. Beizen, abrasives Wasserstrahlen, Sandstrahlen oder Partikelauflagerungen. Anschließend können die Polymere, die die Oberflächenschicht ausbilden, aufgebracht werden. Dies kann z.B. silikon-basierte Polymere sein, aber auch Polyurethane, wie z.B. Teflon, Polypropylen, PEEK, PEAK, HDPE, LDPE, UHMWPE oder Polyamide sein. Diese können z.B. durch chemische Beschichtungs- oder Coating-Prozesse einfach aufgebracht werden. Die Polymere können dafür auch nachfolgend vernetzt werden (chemisches cross-linking), damit kein mechanischer Abrieb beim Anpassen und Einbringen erfolgt.

Eine weitere Möglichkeit stellt das polymer basierte Verkleben von Schraubenkopf und Schraubendreher dar. Dabei werden das Implantat mit der Schraubenklinge durch polymere, bioresorbierbare chemische Substanzen, wie z.B. PDLLA, PCL, PLGA, PLA und PGA direkt miteinander verbunden und ohne weitere Aufnahme eingebracht. Anschließend werden die Schraubenklinge und etwaige Implantate entweder mechanisch oder thermisch getrennt, so dass das Implantat im Körper verbleiben kann und der Teil des Instrumentes, z.B. die Schraubenklinge, weggeworfen / verworfen werden kann. Hierbei ist sichergestellt, dass keine direkte mechanische Interaktion von Werkzeug/Instrument und Implantat auftritt.

Wenn in dem Set ein durch die Schraube zu befestigendes vorzugsweise biodegradierbares und/oder metallisches Implantat enthalten ist, so kann mit einer einzigen fallbezogenen Packungseinheit eine zielgerichtete Versorgung am Patienten für die Operation bewirkt werden.

Standardsysteme mit Klingen und Einsätzen mit bekannten Schnittstellen, wie z.B. Torx, Kreuzschlitz, Innenmehrkant und (Einfach-)Schlitz, können wesentlich verbessert werden. So kann die Schnittstelle aus unterschiedlichen Polymeren oder einem einzigen Polymer bestehen. Die Schnittstelle kann unterschiedliche Keramiken aufweisen oder unterschiedliche Komposit-Materialien besitzen. Insbesondere faserverstärkte Materialien bieten sich für die Schnittstelle an.

Die Schnittstelle kann aus unterschiedlichen oben aufgeführten Materialien in Kombination mit einer Beschichtung oder Beschichtungen oder Oberflächenmodifikationen verbessert werden. Insbesondere der Einsatz von einer inhärenten zwei- oder dreidimensionalen Hohlstruktur bietet sich an. Es kann eine Funktion zur Selbsthaltung des Verbindungselementes genutzt werden. Ebenfalls ist eine Sicherstellung eines Eindreh- bzw. Ausdrehmomentes, bspw. über die Konfiguration des Schraubendrehers als Drehmomentenschlüssel, realisierbar.

Die Schnittstelle kann mit oder ohne Funktion zur einfachen Aufnahme eines aus biodegradierbaren metallischen, sowie resorbierbaren bzw. nicht resorbierbaren Polymeren, Keramiken und deren Komposite bestehenden Implantates ausgestattet sein. Es kann eine Funktion zur einfachen Entnahme aus der Verpackung vorgehalten sein. Ferner können Schnittstellen mit oder ohne Funktion für eine spezifische Energieübertragung zwischen dem Hilfsmittel, dem Werkzeug oder eine strukturierte Vorrichtung und der spezifischen Aufnahme oder dem biodegradierbaren metallischen Implantat selbst ausgestaltet sein. Eine winkelunabhängige Ausrichtung, unter Einsatz spezieller Geometrien ist möglich. Letztlich wird ein biodegradierbares metallisches Implantat bei minimalem Zeit- und Kraftaufwand fixierbar.

Für die Spitzen des Schraubendrehers, die auch als Klingen bezeichnet werden können, haben sich folgende Materialien bewährt. Polymere, insbesondere mit einer Härte nach Rockwell (entsprechend Norm ISO 2039-2) zwischen R30 bis R125, M50 bis M200, E50 bis E200. Dieses Polymer kann auch eine Shore-Härte von 20 Shore A bis 100 Shore A, 20 Shore B bis 100 Shore B, 20 Shore C bis 100 Shore C, 20 Shore D bis 100 Shore D besitzen. Bei Einsatz einer Keramik in besagtem Bereich, sollte eine Härte nach Vickers von 800 bis 2.000 vorgehalten sein. Für faserverstärkte Materialien in dem besagten Bereich sollte eine Härte nach Rockwell, siehe wiederum Norm ISO 2039-2, von R30 bis R150 bzw. M50 bis M250 und E50 bis E250 eingestellt werden.

Die oben aufgeführten Materialien können auch in Kombination mit einer Beschichtung Beschichtungen und/oder einer Oberflächenmodifikation oder Oberflächenmodifikationen genutzt werden. Bei Polymeren sollte dann nach Norm ISO 2039-2 gemäß Rockwell eine Härte von R30 bis R200, bzw. M50 bis M300, bzw. E50 bis E300 vorliegen, also nach Shore gemessen 20 Shore A bis 200 Shore A, 20 Shore B bis 200 Shore B, 20 Shore C bis 200 Shore C bzw. 20 Shore D bis 200 Shore D. Bei Keramiken sollte eine Härte nach Vickers von ca. 800 bis ca.3.000 vorliegen. Für faserverstärkte Materialien nach Rockwell, siehe besagte Norm ISO 2039-2, sollte eine Härte von R30 bis R150 bzw. M50 bis M250 bzw. E50 bis E250 eingestellt werden. Für Metalle (legierte und unlegierte) gibt es nach der DIN-Norm 50150 hier auch heranziehbare Umrechnungstabellen. Siehe bspw. auch im Internet unter http://www.chemie.de/lexikon/H%c3%a4rte.html#h.c3.a4rtepr.c3.bcfung nach Rockwel l.

Die Erfindung wird nachfolgend mit Hilfe einer Zeichnung näher erläutert. Dabei sind mehrere Ausführungsbeispiele näher dargestellt, welche abwandelbar sind. Es zeigen:
- Fig. 1: ein erfindungsgemäßes Implantationsset in einer Seitenansicht mit einem Schraubendreher, der auf eine Schraube mit seiner Spitze aufgesetzt ist,
- Fig. 2: eine Vergrößerung des Bereiches II aus Fig. 1,
- Fig. 3: eine Vergrößerung des Bereiches III aus Fig. 2,
- Fig. 4: eine Vergrößerung des Bereiches IV aus Fig. 3
- Fig. 5: eine perspektivische Darstellung des in Fig. 4 dargestellten Bereiches,
- Fig. 6: einen teilweise im Längsschnitt dargestellten Koppelbereich zwischen dem Schraubendreher und der Schraube,
- Fig. 7: eine perspektivische Darstellung der Schraube und der nur im Bereich der Spitze / Klinge des Schraubendrehers dargestellten Schraubendrehers in einer Seitenansicht,
- Fig. 8: die Bauteile aus Fig. 7 in einer perspektivischen Darstellung,
- Fig. 9: eine perspektivische Ansicht distal auf die Spitze des Schraubendrehers,
- Fig. 10: eine perspektivische Ansicht des Schraubenkopfbereiches mit seiner dort vorhandenen Werkzeugaufnahme nach Art einer Torx-Vertiefung zum Realisieren eines Schraubenantriebs,
- Fig. 11: einen Längsschnitt durch den Schraubendreher des Implantationssets aus Fig. 1 zum Realisieren einer zwei- oder dreidimensionalen Hohlstruktur,
- Fig. 12: der operative Einsatz des Implantationssets zusammen mit einem biodegradierbaren metallischen Implantat bei winkelunabhängiger Ausrichtung,
- Fig. 13: eine Vergrößerung eines Verkeilungsbereiches der Schraube mit dem Implantat, und
- Fig. 14: eine Seitenansicht zur Symbolisierung eines Winkelversatzes von +/-ca. 15°, innerhalb welchem ein Verkeilen des Außengewindes der Schraube mit einem Innengewinde des Implantats erreicht wird.

Die Figuren sind lediglich schematischer Natur und dienen nur dem Verständnis der Erfindung. Die gleichen Elemente sind mit denselben Bezugszeichen versehen. Die Merkmale der einzelnen Ausführungsbeispiele können auch untereinander ausgetauscht werden.

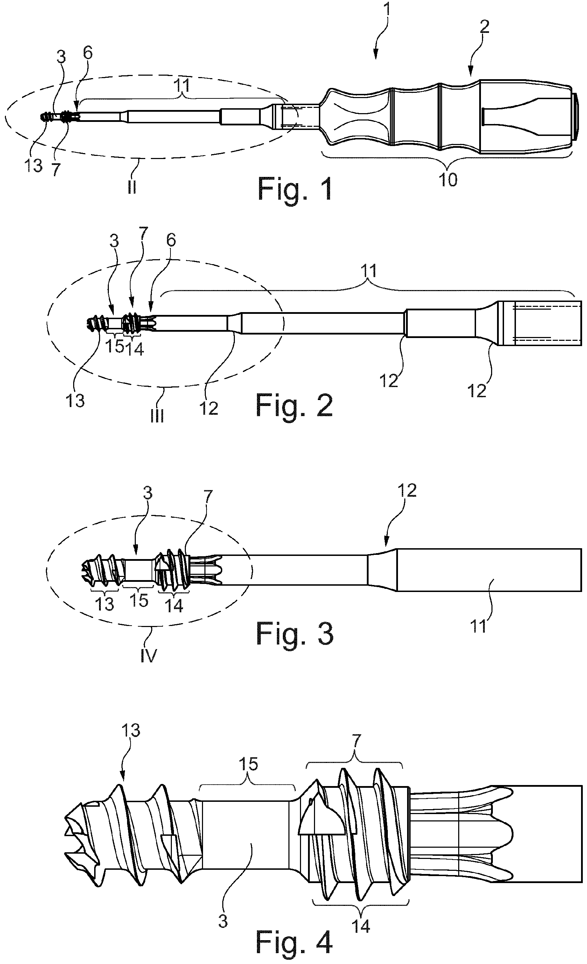

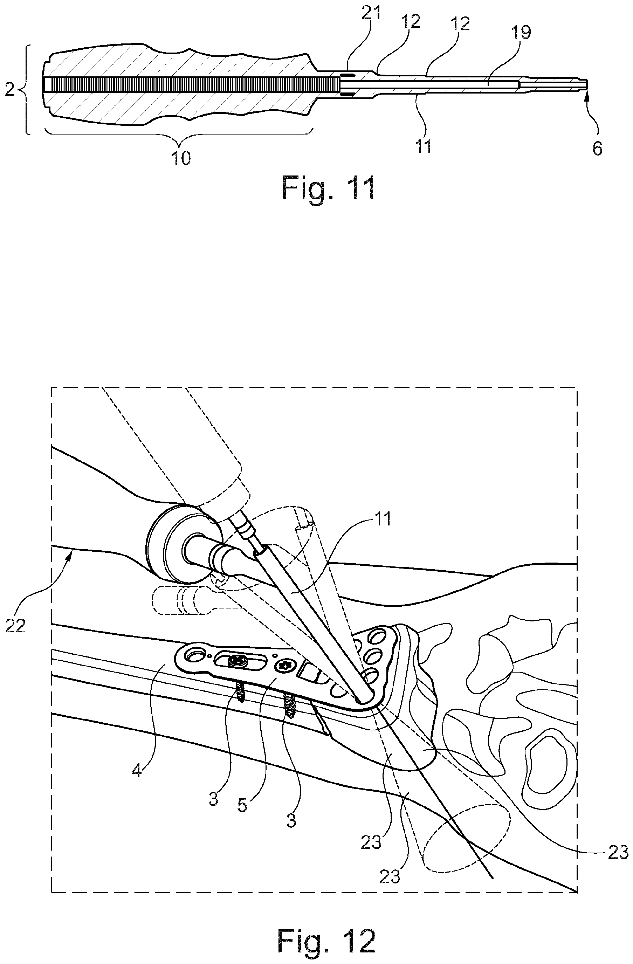

In Fig. 1 ist ein erstes erfindungsgemäßes Implantationsset 1 dargestellt. Dieses weist einen Schraubendreher 2 auf. Der Schraubendreher 2 kontaktiert dabei eine (Knochen-) Schraube 3. Die Schraube 3 ist zum Einschrauben in einen Knochen 4, siehe Fig. 12, vorgesehen und soll dann ein Implantat 5 am Knochen 4 befestigen.

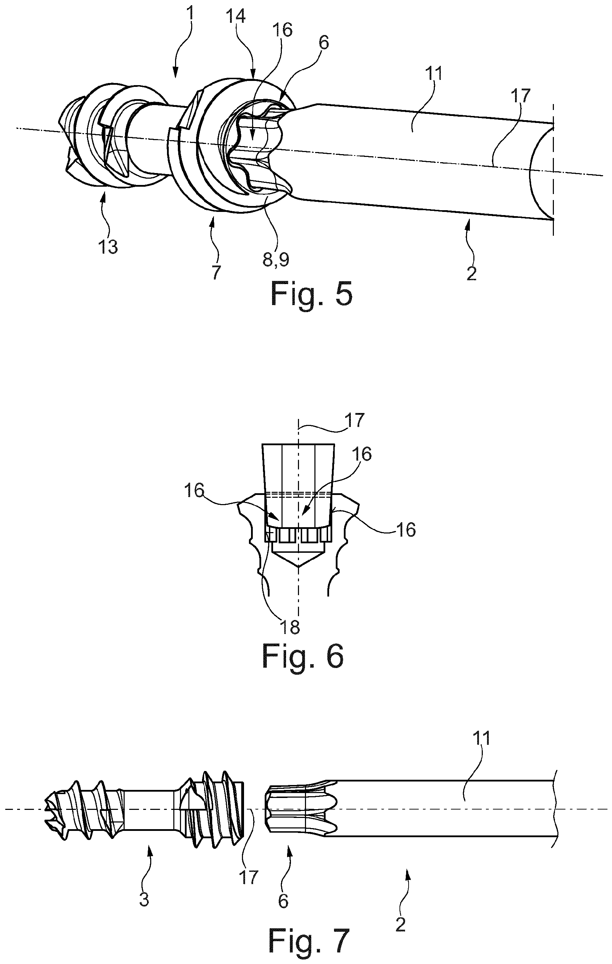

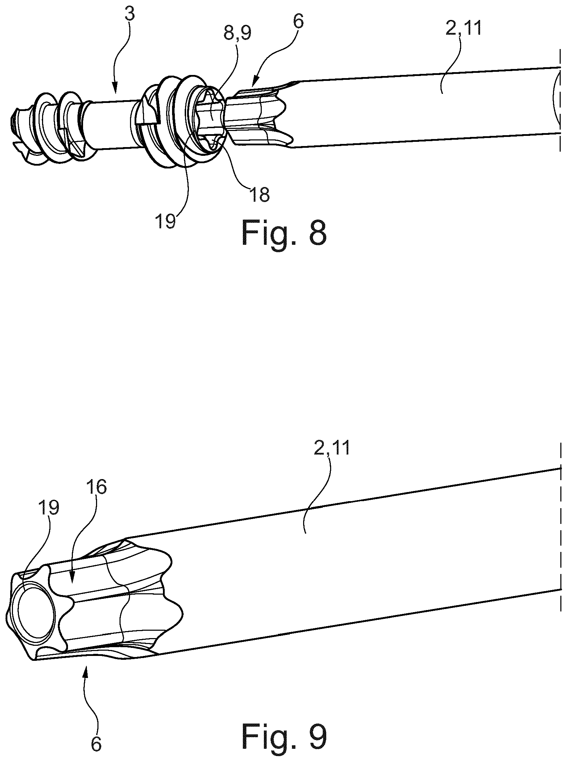

Zurückkommend auf Fig. 1 sei das Augenmerk auf eine Spitze 6 des Schraubendrehers 2 gerichtet. Diese Spitze 6 greift in einem drehmomentübertragendem Zustand in eine Werkzeugaufnahme 8 der Schraube 3, nämlich an einem schraubenkopfseitigen Ende. Die Werkzeugaufnahme 8 der Schraube 3 ist als Vertiefung 9 ausgebildet. Das Zusammenspiel aus Spitze 6 und Vertiefung 9 wird bei Betrachtung der Figuren 6 und 8 offensichtlich.

Zurückkommend auf Fig. 1 fällt auf, dass zwischen der distal endenden Spitze 6 und einem Griff / Griffbereich 10 des Schraubendrehers ein Schaft 11 dazwischengeschaltet ist. Der Schaft 11 weist Außenstufen 12 auf. Es sind drei Außenstufen 12 vorhanden.

Am schraubenkopffernen Ende der Schraube 3 ist ein Knochengewinde / Außengewinde 13 vorhanden. Ein Implantatkontakt(außen)gewinde 14 ist im Schraubenkopfbereich vorhanden. Es kann dieselbe oder eine andere Steigung wie das Knochengewinde / Außengewinde 13 besitzen. Zwischen dem Knochengewinde / Außengewinde 13 und dem auf der Außenseite vorhandenen Implantatkontaktgewinde 14 ist ein gewindefreier Bereich 15 vorhanden. Dies ist besonders gut in den Fign. 2 bis 4 zu erkennen.

In Fig. 5 ist das Eintauchen einer eine regelmäßige achtfach Torx-Konfiguration auf der Außenseite besitzenden Spitze 6 des Schraubendrehers 2 in eine dazu passende Vertiefung 9 der Werkzeugaufnahme 8 im Schraubenkopf 7 der Schraube 3 dargestellt.

Dass die Spitze 6 des Schraubendrehers 2 Werkzeugaufnahmekontaktflächen 16 besitzt, ist der Figur 6 zu entnehmen. Diese Werkzeugaufnahmekontaktflächen 16 sind zu einer Längsachse 17 des Schraubendrehers 2 bzw. der Schraube 3 geneigt. Es stellt sich der Winkel α ein. Als besonders bevorzugter Wert für α hat sich 5° herausgestellt, um eine Selbsthaltung beim dargestellten Torx-Einsatz zu erreichen. Die Werkzeugaufnahmekontaktflächen 16 kontaktieren dabei Schraubendreherspitzenkontaktflächen 18 in der Schraube 3.

In den Figuren 7 und 8 ist die Schraube 3 kurz vor einer Kontaktierung durch die Spitze 6 dargestellt. Wie in den Figuren 8 und besonders 9 gut zu erkennen, weist sowohl die Schraube 3, als auch der Schraubendreher 2 ein Durchgangsloch 19 auf.

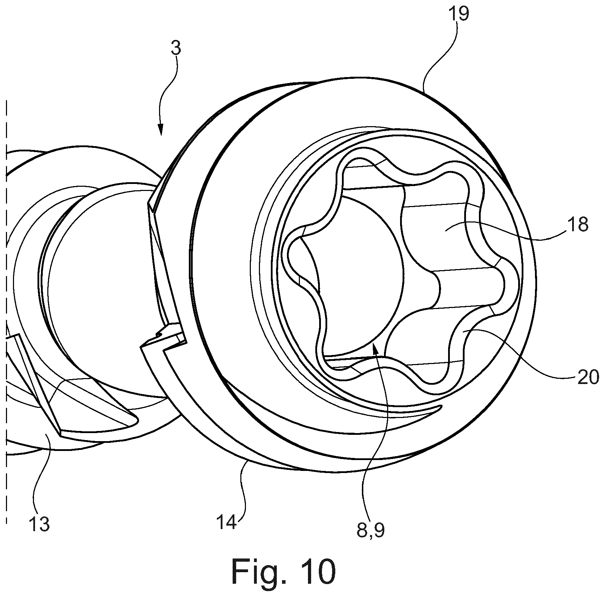

Schraubenseitig ist am Eingang der Vertiefung 9 eine Anfassung 20 vorhanden. Dies ist in Fig. 10 gut zu erkennen.

Dass der Schraubendreher 2 im Durchgangsloch 19 mehrere Stufen 21 besitzt ist besonders gut in Fig. 11 zu erkennen. Der Griff 10 ist als Wegwerfartikel aus einem Kunststoffspritzgussmaterial hergestellt, wohingegen der Schaft 11 aus gehärtetem Stahl bestehen kann, wenn bspw. die Schraube aus Titan gefertigt ist. Die Schraube 3 kann auch eine keramische Beschichtung besitzen. Die Spitze 6 kann aus einem dazu weicheren Material bestehen und koppelbar am Schaft 11 angebracht werden. Dass das Durchgangsloch 10 im Bereich des Griffs 10 größer ist, als im Bereich der Spitze 6, zieht eine gute Reinigungswirkung nach sich, wenn statt Nutzung des Griffs 10 als Wegwerfartikel, dieser wiederverwendet werden soll und daher gereinigt werden muss.

Ein winkelunabhängiger Einsatz eines Werkzeugs ist in Fig. 12 angedeutet. Allerdings ist dort ein Bohrwerkzeug 22 mit einem Bohrer 23 im Einsatz. Es sind unterschiedliche Positionen des Bohrwerkzeugs 22 und des Bohrers 23 angedeutet. Anstelle des Bohrwerkzeugs 22 ist der Schraubendreher winkelähnlich / lageähnlich oder -identisch einsetzbar.

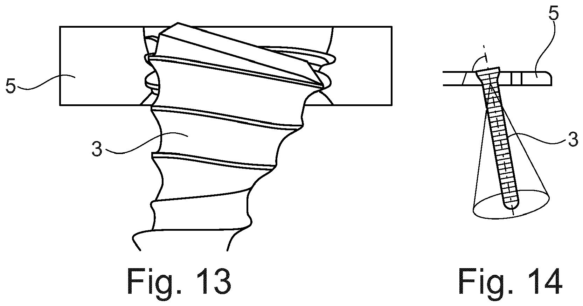

Die Kombination aus dem Implantat 5 und der (Knochen-)Schraube 3 ist der Fig. 13 gut zu entnehmen, wie auch der Fig. 14. Allerdings ist hier die Schraube 3 etwas anders ausgebildet, als die in den vorher dargestellten Ausführungsbeispielen genutzte Schraube 3.

Als Härte für eine Schraube 3 nach Brinell bieten sich Werte von 70 bis 90 an. Die Spitze 6, die auch als Klinge oder Klingenspitze bezeichnet werden kann, kann insbesondere auch als Einmalartikel gefertigt sein, insbesondere aus Polymer und sollte dann eine Härte, z.B. unter Einsatz von PEEK 1000 von M105 nach ISO 2039-2 besitzen oder bei Einsatz von PEEK mit Karbonfasern von M105 nach DIN ISO 2039-2 besitzen. Bei Einsatz von Keramik sollte eine hohe Härte vorherrschen. Zirkoniumoxid oder Aluminiumoxid bietet sich dabei alternativ oder zusätzlich an. Oberflächenmodifikationen, bspw. nach Art von Oxidierungen, Nitrilisierungen und PVD-Beschichtungen sind denkbar. Ein Winkel zur Selbsthaltung ist mit ca. 5° auf jeder Seite zur Längsachse gemessen (etwa bei Verwendung von Torx) oder 1° auf jeder Seite gemessen oder 5° auf jeder Seite gemessen denkbar. Summenwinkel von 5° oder 2 bis 10° sind somit die Folge.

### Bezugszeichenliste

- 1: Implantationsset
- 2: Schraubendreher
- 3: (Knochen-)Schraube
- 4: Knochen
- 5: Implantat
- 6: Spitze
- 7: Schraubenkopf
- 8: Werkzeugaufnahme
- 9: Vertiefung
- 10: Griff / Griffbereich
- 11: Schaft
- 12: Außenstufe
- 13: Knochengewinde / Außengewinde
- 14: Implantatkontaktgewinde
- 15: gewindefreier Bereich
- 16: Werkzeugaufnahmekontaktfläche der Spitze des Schraubendrehers
- 17: Längsachse
- 18: Schraubendreherspitzenkontaktfläche des Schraubenkopfes der Schraube
- 19: Durchgangsloch
- 20: Anfasung
- 21: Stufe
- 22: Bohrwerkzeug
- 23: Bohrer

## Patentansprüche

1. Implantationsset (1) mit einem Werkzeug und zumindest einem dazu passenden Knochenkontaktierungsorgan, das als eine Vorrichtung zum direkten Kontaktieren eines Knochens ausgelegt ist, wobei das Knochenkontaktierungsorgan in einem zum Kontaktiertwerden durch das Werkzeug vorgesehenen Bereich eine größere Härte besitzt als das Werkzeug in einem zum Kontaktieren des Knochenkontaktierungsorgan vorgesehenen Bereich, wobei das Werkzeug als ein Schraubendreher (2) ausgebildet ist und das Knochenkontaktierungsorgan als eine ein Implantat ausbildende Schraube (3), die zum Knochenkontaktierungsorgan passt, zum Eindrehen in einen Knochen (4), ausgebildet ist, wobei die Schraube (3) einerseits einen Schraubenkopf (7) und andererseits ein Gewinde (13, 14) besitzt, wobei der Schraubenkopf (7) eine Werkzeugaufnahme (8) besitzt, die geometrisch an die Außenkontur einer Spitze (6) des Schraubendrehers (2) angepasst ist, wobei der Schraubenkopf (7) zumindest im Bereich der Werkzeugaufnahme (8) eine größere Härte besitzt, als zumindest der zum Kontaktieren der Werkzeugaufnahme (8) vorgesehene Teil der Spitze (6) des Schraubendrehers (2), und wobei die Werkzeugaufnahme (8) in Längsrichtung der Schraube (3) ausgerichtete Schraubendreherspitzenkontaktflächen (18) besitzt, wohingegen Werkzeugaufnahmekontaktflächen (16) an der Spitze (6) des Schraubendrehers (2) zur Längsrichtung (17) der Schraube (3) geneigt ausgerichtet sind, **dadurch gekennzeichnet, dass** alle zum Kontaktieren der Schraubendreherspitzenkontaktflächen (18) der Schraube (3) vorgesehenen Werkzeugaufnahmekontaktflächen (16) des Schraubendrehers (2) zwischen ca. 2,5° bis ca. 7,5° zur Längsachse (17) des Schraubendrehers (2) geneigt sind.

2. Implantationsset (1) nach Anspruch 1, **dadurch gekennzeichnet, dass** die Werkzeugaufnahme (8) als Vertiefung (9) ausgebildet ist und/oder die Werkzeugaufnahme (8) eine Schlitz-, Kreuzschlitz-, Inbus- oder Torx-Geometrie besitzt und der Schraubendreher (2) an der Spitze (6) dazu passend ausgebildet ist.

3. Implantationsset (1) nach Anspruch 2, **dadurch gekennzeichnet, dass** die Geometrien und die Ausmaße der Vertiefung (9) im Schraubenkopf (7) und der als Vorsprung ausgebildeten Spitze (6) des Schraubendrehers (2) so aufeinander angepasst sind, dass ein Selbsthalten der Schraube (3) auf der Spitze (6) beim Eingreifen der Spitze (6) in die Vertiefung (9) erzwungen ist.

4. Implantationsset (1) nach einem der Ansprüche 1 bis 3, **dadurch gekennzeichnet, dass** die Schraube (3) und der Schraubendreher (2) jeweils ein Durchgangsloch (19) besitzen.

5. Implantationsset (1) nach Anspruch 4, **dadurch gekennzeichnet, dass** die Durchgangslöcher (19) der Schraube (3) und des Schraubendrehers (2) im gegenseitigen Kontaktbereich im Querschnitt kreisrund und gleich groß ausgebildet sind.

6. Implantationsset (1) nach einem der Ansprüche 4 oder 5, **dadurch gekennzeichnet, dass** das Durchgangsloch (19) im Schraubendreher (2) mehrfach gestuft ausgebildet ist.

## Claims

1. An implantation set (1) comprising a tool and at least one bone contact member which is adapted thereto and is designed as a device for direct contact with a bone, wherein the bone contact member has a greater hardness in a region intended for contact by the tool than the tool in a region intended for contact with the bone contact member, wherein the tool is designed as a screwdriver (2) and the bone contact member is designed as a screw (3) forming an implant and matching the bone contact member for screwing into a bone (4), wherein the screw (3) has on the one hand a screw head (7) and on the other hand a thread (13, 14), wherein the screw head (7) has a tool mount (8) which is geometrically adapted to the outer contour of a tip (6) of the screwdriver (2), wherein the screw head (7) has a greater hardness at least in the region of the tool mount (8) than at least the part of the tip (6) of the screwdriver (2) provided for contacting the tool mount (8), and wherein the tool mount (8) has screwdriver tip contact surfaces (18) aligned in the longitudinal direction of the screw (3), whereas tool mount contact surfaces (16) at the tip (6) of the screwdriver (2) are aligned so as to be inclined relative to the longitudinal direction (17) of the screw (3) **characterized in that** all tool mount contact surfaces (16) of the screwdriver (2) provided for contacting the screwdriver tip contact surfaces (18) of the screw (3) are inclined between approximately 2.5° to approximately 7.5° relative to the longitudinal axis (17) of the screwdriver (2).

2. The implantation set (1) according to claim 1, **characterized in that** the tool mount (8) is designed as a recess (9) and/or the tool mount (8) has a slot, cross-slot, hexagonal or Torx geometry and the screwdriver (2) at the tip (6) has a matching design.

3. The implantation set (1) according to claim 2, **characterized in that** the geometries and the dimensions of the recess (9) in the screw head (7) and the tip (6) of the screwdriver (2), which is designed as a projection, are adapted to one another such that a self-retaining effect of the screw (3) on the tip (6) is enforced when the tip (6) engages in the recess (9).

4. The implantation set (1) according to claim 1 to 3, **characterized in that** the screw (3) and the screwdriver (2) each have a through-hole.

5. The implantation set (1) according to claim 4, **characterized in that** the through-holes (19) of the screw (3) and the screwdriver (2) are of circular cross-section and have the same size in the mutual contact region.

6. The implantation set (1) according to claim 4 or 5, **characterized in that** the through-hole (19) in the screwdriver (2) has a multi-step design.

## Revendications

1. Ensemble d'implantation (1) comprenant un outil et au moins un organe de mise en contact avec l'os s'adaptant audit outil et réalisé sous forme de dispositif permettant une mise en contact directe avec un os, dans lequel l'organe de mise en contact avec l'os présente, dans une région prévue pour être mise en contact par l'outil, une dureté supérieure à celle de l'outil dans une région prévue pour être mise en contact avec l'organe de mise en contact avec l'os, dans lequel l'outil est réalisé sous la forme d'un tournevis (2) et l'organe de mise en contact avec l'os est réalisé sous la forme d'une vis (3) qui forme un implant, s'adapte à l'organe de mise en contact avec l'os et permet un vissage dans un os (4), dans lequel la vis (3) présente d'une part une tête de vis (7) et d'autre part un filetage (13, 14), dans lequel la tête de vis (7) présente un logement d'outil (8) géométriquement adapté au contour extérieur d'une pointe (6) du tournevis (2), dans lequel la tête de vis (7) présente, au moins dans la région du logement d'outil (8), une dureté supérieure à au moins celle de la partie de la pointe (6) du tournevis (2) prévue pour une mise en contact avec le logement d'outil (8), et dans lequel le logement d'outil (8) présente des surfaces de contact de pointe de tournevis (18) orientées dans la direction longitudinale de la vis (3), tandis que des surfaces de contact de logement d'outil (16) situées au niveau de la pointe (6) du tournevis (2) sont orientées de manière inclinée par rapport à la direction longitudinale (17) de la vis (3), **caractérisé en ce que** toutes les surfaces de contact de logement d'outil (16) du tournevis (2) prévues pour être mises en contact avec les surfaces de contact de pointe de tournevis (18) de la vis (3) sont inclinées dans une plage comprise entre environ 2,5° et environ 7,5° par rapport à l'axe longitudinal (17) du tournevis (2).

2. Ensemble d'implantation (1) selon la revendication 1, **caractérisé en ce que** le logement d'outil (8) est réalisé sous la forme d'un renfoncement (9) et/ou le logement d'outil (8) présente une géométrie fendue, cruciforme, Allen ou Torx, et le tournevis (2) est réalisé de manière adaptée à celle-ci au niveau de la pointe (6).

3. Ensemble d'implantation (1) selon la revendication 2, **caractérisé en ce que** les géométries et les dimensions du renfoncement (9) situé dans la tête de vis (7) et celles de la pointe (6), réalisée sous la forme d'une saillie, du tournevis (2) sont adaptées les unes aux autres de sorte qu'un maintien automatique de la vis (3) sur la pointe (6) est imposé lors de la mise en prise de la pointe (6) dans le renfoncement (9).

4. Ensemble d'implantation (1) selon l'une quelconque des revendications 1 à 3, **caractérisé en ce que** la vis (3) et le tournevis (2) présentent respectivement un trou traversant (19).

5. Ensemble d'implantation (1) selon la revendication 4, **caractérisé en ce que** les trous traversants (19) de la vis (3) et du tournevis (2) sont de section circulaire et de même dimension dans la région de contact mutuel.

6. Ensemble d'implantation (1) selon l'une quelconque des revendications 4 ou 5, **caractérisé en ce que** le trou traversant (19) présente plusieurs étagements au sein du tournevis (2).
